# EUROPEAN PATENT SPECIFICATION

(11) **EP 2 542 061 B1**
(45) Date of publication and mention of the grant of the patent: **12.06.2019**
(21) Application number: 11703791.1
(22) Date of filing: 02.02.2011
(51) Int. Cl.: A01N 37/16, A01N 25/22, A61L 2/18, A61L 2/10, A01P 1/00, A01N 59/00

(54) **MOLYBDATE-FREE STERILIZATION COMPOSITION CONTAINING PERACETIC ACID**
MOLYBDATFREIE STERILISATIONSZUSAMMENSETZUNG MIT PERESSIGSÄURE
COMPOSITION STÉRILISANTE SANS MOLYBDATE CONTENANT DU L'ACIDE PERACETIQUE

(30) Priority: 05.03.2010 US 718078
(43) Date of publication of application: 09.01.2013
(73) Proprietor: American Sterilizer Company, Mentor, OH 44060-1834 (US)
(72) Inventor: FRANCISKOVICH, Phillip, P., Concord Ohio 44077 (US); ROSENHAMER, Donald, G., Garfield Heights Ohio 44125 (US); FIX, Kathleen, A., Willoughby Ohio 44094 (US); HALL, Dana, St. Louis Missouri 63146 (US)
(74) Representative: Scholz, Volker
(86) International application number: PCT/US2011/023429
(87) International publication number: WO 2011/109136

(56) References cited:
- EP-A1- 1 226 835
- EP-A1- 1 252 819
- US-A- 5 624 634
- US-A1- 2007 031 464
- US-A1- 2009 005 590
- US-A1- 2009 061 017
- US-A1- 2009 178 981
- US-A1- 2009 238 719
- US-B1- 6 589 565

## Description

### Technical Field

This invention relates to a method for sterilizing a medical, dental, pharmaceutical, veterinary or mortuary instrument at the temperature from 20 to 80°C using peracetic acid based liquid sterilant, free of molybdate, nonylphenol ethoxylate and an antifoaming agent, said method characterised by supplying components (A) and (B) (as defined in claims) separately and dispersing the supplied components (A) and (B) in water at the time the sterilizing is conducted.

### Background

Medical, dental, pharmaceutical, veterinary or mortuary instruments and devices that are exposed to blood or other body fluids require sterilizing or disinfecting between each use. Liquid sterilizing or disinfecting systems are used to clean and decontaminate instruments and devices that cannot withstand the high temperatures of steam sterilization.

US 2009/238719 discloses methods and agents for cleaning and disinfecting fragile medical appliances.

EP 1 226 835 discloses a method of disinfecting premises by thermos-spraying and aqueous solution of peracetic acid.

US 5,624,634 discloses a peracid composition for medical disinfection.

US 6,589,565 discloses non-corrosive sterilant compositions.

US 2007/031464 discloses sterilant compositions and systems.

EP 1 252 819 discloses a disinfecting composition for ophthalmologic devices.

US 2009/061017 discloses shelf stable, reduced corrosion, ready to use peroxycarboxylic acid antimicrobial compositions.

US 2009/005590 discloses the production of peracids using an enzyme having perhydrolysis activity.

### Summary

Problems of the prior art have been overcome in accordance with the subject-matter of the independent claim. Preferred embodiments result from the sub-claims.

In detail, the invention relates to a method for sterilizing a medical, dental, pharmaceutical, veterinary or mortuary instrument at a temperature of 20°C to 80°C using a liquid sterilant, comprising supplying components (A) and (B) separately and dispersing the supplied components (A) and (B) in water at the time the sterilizing is conducted, wherein:
component (A) consists essentially of: 15 to 45% by weight peracetic acid; 34 to 62% by weight acetic acid; 6.5 to 32% by weight hydrogen peroxide; and 0.5 to 2% by weight sulfuric acid; and
component (B) consists essentially of: 35 to 98% by weight of a buffer, wherein the buffer is selected from the group consisting of an alkali metal phosphate, an alkali metal carbonate, or a mixture thereof; 0.5 to 35% by weight of an anticorrosive agent; and 0.1 to 60% by weight of a chelator; and
wherein the weight ratio of component (A) to component (B) is from 0.1 to 1.3; and wherein the liquid sterilant composition comprises molybdate, nonylphenol ethoxylate, and an antifoaming agent, respectively in an amount of 0.01% by weight or less.

In one embodiment, the concentration of component (A) in the aqueous liquid sterilant is in the range from 0.5 to 10 g/l, and the concentration of component (B) in the aqueous liquid sterilant is in the range from 3.6 to 18 g/l, and the liquid sterilant has a pH in the range from 2 to 11, or from 5.5 to 7.

In a further embodiment, the method further comprises contacting the medical, dental, pharmaceutical, veterinary or mortuary instrument with the liquid sterilant.

In another embodiment, the exposure time of the medical, dental, pharmaceutical, veterinary or mortuary instrument is in the range from 0.5 to 240 min, or from 2 to 60 min.

### Brief Description of the Drawings

In the annexed drawings, like references indicate like parts and features.
Fig. 1 is a flow sheet showing a sterilization process that may be used in accordance with the invention.
Fig. 2 is a flow sheet showing a filtration system that may be used with the sterilization process illustrated in Fig. 1.
Fig. 3 is a plot showing the concentration of peracetic acid (PAA) over time for a liquid sterilant based on the formulations disclosed in the Examples, these formulations being referred to as the "Example 1" formulation and the "Example C-1" formulation.
Fig. 4 is a plot of pH over time for a liquid sterilant based on the Example 1 formulation and the Example C-1 formulation.
Fig. 5 is a plot showing comparative resulting corrosivity over time for a liquid sterilant based on the Example 1 formulation and the Example C-1 formulation.
Fig. 6 is a plot showing chelation capacity needed for a liquid sterilant based on the Example 1 formulation and the Example C-1 formulation.
Fig. 7 is a plot showing time of exposure to a sterilant needed to sterilize an article using a liquid sterilant based on the Example 1 formulation (6 minutes) and the Example C-1 formulation (12 minutes).

### Detailed Description

All ranges and ratio limits disclosed in the specification and claims may be combined. It is to be understood that unless specifically indicated, references to "a," "an" and/or "the" may include one or more than one, and that reference to an item in the singular may also include the item in the plural.

The term "sterilization" refers to rendering a substance incapable of reproduction, metabolism and/or growth. The term "sterilization" includes microbial deactivation. While sterilization is often taken to refer to a total absence of living organisms, the term may be used herein to refer to a substance free from living organisms to a degree agreed to be acceptable. Unless otherwise indicated, the term "sterilization" may be used herein to also refer to processes less rigorous than sterilization, for example, disinfection, sanitization, decontamination, cleaning, and the like. Variations of the term "sterilization," such as sterilant, sterilizing, etc., may also be used herein to refer to and encompass related variants associated with sterilization processes as well as processes less rigorous than sterilization (e.g., disinfectant, disinfecting, etc.).

The inventive composition may comprise a liquid sterilant which may be made by dispersing or dissolving components (A) and (B) in water. The water may be taken from any source. The water may comprise deionized water, tap water, processed tap water, or the like.

Component (A) consists essentially of: 15 to 45% by weight peracetic acid; 34 to 62% by weight acetic acid; 6.5 to 32% by weight hydrogen peroxide; and 0.5 to 2% by weight sulfuric acid; and component (B) consists essentially of: 35 to 98% by weight of a buffer, wherein the buffer is selected from the group consisting of an alkali metal phosphate, an alkali metal carbonate, or a mixture thereof; 0.5 to 35% by weight of an anticorrosive agent; and 0.1 to 60% by weight of a chelator; and wherein the weight ratio of component (A) to component (B) is from 0.1 to 1.3; and wherein the component (B) comprises 0.01% by weight or less of molybdate, nonylphenol ethoxylate, and an antifoaming agent respectively.

The alkali metal may comprise sodium or potassium. The buffer may comprise one or more of monosodium phosphate, disodium phosphate, trisodium phosphate, monopotassium phosphate, dipotassium phosphate, tripotassium phosphate, sodium carbonate, or a mixture of two or more thereof. Disodium phosphate may be preferred.

The anticorrosive agent may comprise benzotriazole, a sodium salt of benzotriazole, tolyltriazole, a sodium salt of tolyltriazole, or a mixture of two or more thereof. Sodium benzotriazole may be preferred. A commercially available sodium benzotriazole that may be used is available under the trade designation Cobratec 40S which is believed to be a 40% by weight aqueous solution of sodium benzotriazole.

The chelator may comprise ethylenediaminetetraacetic acid, hydroxyethylidenediphosphonic acid, a sodium salt of either of these acids, or a mixture of two or more thereof. A preferred sodium salt of ethylenediaminetetraacetic acid may be ethylenediaminetetraacetic acid, tetrasodium salt, tetrahydrate. A commercially available ethylenediaminetetraacetic acid, tetrasodium salt, tetrahydrate that may be used is available from Akzo Nobel under the trade designation Dissolvine 220-S. Dissolvine 220-S is identified by Akzo

Component (B) may comprise: disodium phosphate; sodium benzotriazole; and ethylenediaminetetraacetic acid, tetrasodium salt, tetrahydrate.

The weight ratio of component (A) to component (B) is from 0.1 to 1.3, or from 0.1 to 1.1, or from 0.15 to 0.9, or from 0.15 to 0.75, or from 0.2 to 0.7. The weight ratio of component (A) to component (B) is from 0.45 to 1.3, or from 0.5 to 1.3, or from 0.6 to 1.3. The weight ratio of peracetic acid to buffer is 0.1 or higher, or from 0.1 to 3, or from 0.3 to 3, or from 0.35 to 1.5.

The concentration of peracetic acid in component (A) is from 15 % to 45 % by weight, from 30 % to 40 % by weight, or 35.5% by weight. The concentration of acetic acid in component (A) is from 34 % to 62 % by weight, or from 40 % to 55% by weight. The concentration of hydrogen peroxide in component (A) is from 6.5 % to 32% by weight. The concentration of sulfuric acid in component (A) is 0.5 to 2.0 % by weight, or 0.75 % to 1.5% by weight. The concentration of water in component (A) may be in the range from 5 % to 60 % by weight, or 10 % to 50% by weight. A commercially available peracetic acid solution which may be used as component (A) is available from FMC Corporation under the trade designation Peracetic Acid 35%. This solution is believed to contain 35.5% by weight peracetic acid, 40% by weight acetic acid, 6.5% by weight hydrogen peroxide, 1 % sulfuric acid, and 17% free water.

Component (B) may comprises from 35 % to 98 % by weight, or 45 % to 95 % by weight, or from 55 to 90% by weight, of the buffer. Component (B) comprises 0.5 to 35 % by weight, or 1 % to 25 % by weight, or from 2 % to 14% by weight, of the anticorrosive agent. Component (B) comprises 0.1 to 60 % y weight, or from 0.3 % to 60 % by weight, or from 0.5 to 55% by weight, of the chelator.

The liquid sterilant made from components (A) and (B) may comprise an aqueous solution wherein the concentration of component (A) may be in the range from 0.5 to 10 g/l or from 1.2 to 3.5 g/l; and the concentration of component (B) is from 3.6 to 18 g/l, or from 5 to 15 g/l. The liquid sterilant may have a pH in the range from 2 to 7, or from 5.5 to 7. The liquid sterilant may be referred to as a low-temperature liquid sterilant. This sterilant is used in the sterilization of medical, dental, pharmaceutical, veterinary and mortuary devices, which cannot be subjected to the high temperatures required for steam sterilization.

An advantage of the inventive composition is that it is characterized by the absence of a molybdate. Another advantage is that the composition is characterized by the absence of a nonylphenol ethoxylate. Another advantage is that the composition is characterized by the absence of an antifoaming agent. Even though the inventive composition is characterized by the absence of one or more of the foregoing materials, it is to be understood that this does not exclude the possibility that trace amounts of one or more of these materials may be present in component (B). The term "trace amount" refers to a concentration of 0.01 % by weight or less, or 0.0001 to 0.1% by weight, relative to the weight of component (B). In one embodiment, component (B) consists of water and three components, namely, a buffer, an anticorrosive agent and a chelator.

The liquid sterilant made from components (A) and (B) may be used in any process for sterilizing articles, including processes for sterilizing articles that cannot withstand the high temperatures required for steam sterilization. The articles that may be sterilized may include medical, dental, pharmaceutical, veterinary or mortuary instruments or devices (e.g., endoscopes). These may be made of a material comprising brass, copper, aluminum, stainless steel, carbon steel, plastic, glass, adhesive, or a combination of two or more thereof. The pH of the liquid sterilant may be in the range from 2 to 11, or from 5.5 to 7. The temperature of the liquid sterilant, when used in a sterilizing process, is in the range from 20 to 80°C, or from 40 to 60°C. The exposure time of the article being sterilized to the liquid sterilant may be in the range from 0.5 to 240 minutes, or from 2 to 60 minutes.

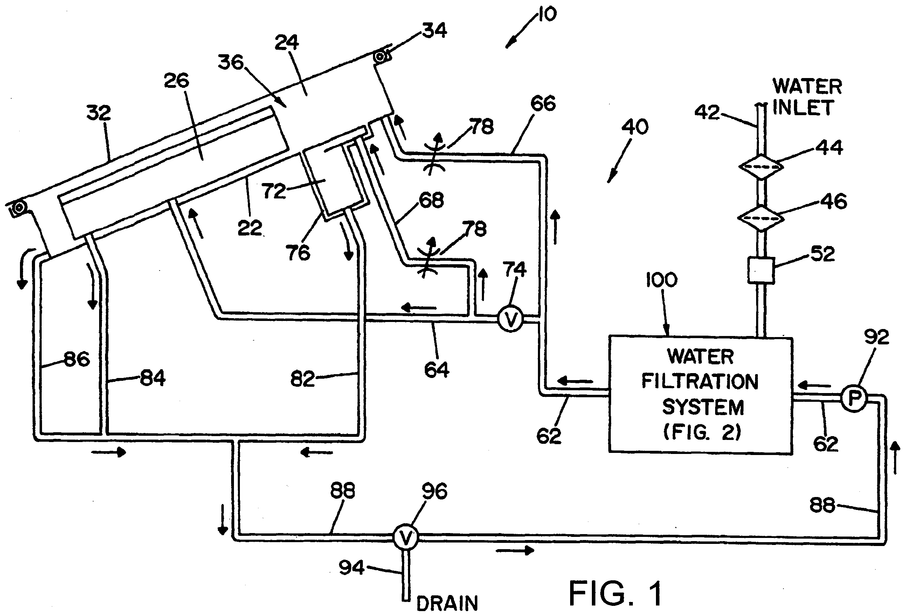

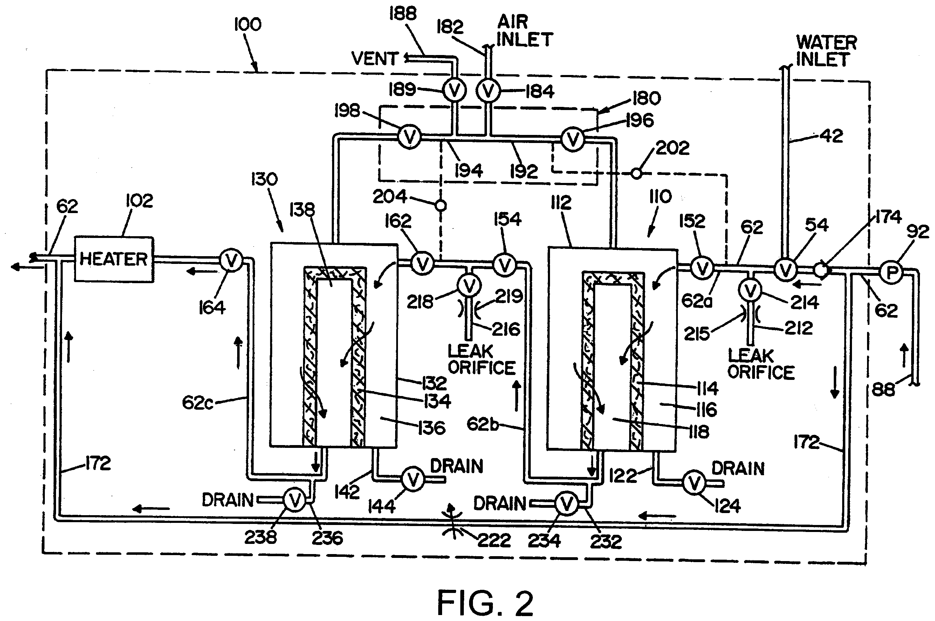

The process may be conducted in any suitable sterilization apparatus. An example of such sterilization apparatus is illustrated in Figs. 1 and 2. Referring to Figs. 1 and 2, sterilization apparatus 10 includes panel 22, which is part of a housing structure (not shown). The panel 22 includes a recess or cavity 24 dimensioned to receive the articles to be sterilized. A tray or container 26 is provided to receive the articles to be sterilized. Container 26 is dimensioned to be received within the recess or cavity 24.

A manually operable lid 32 is movable between an opened position allowing access to cavity 24, and a closed position (shown in Fig. 1) closing or covering cavity 24. A seal element 34 surrounds cavity 24 and forms a fluid-tight, i.e., an air-tight and liquid-tight, seal between lid 32 and panel 22 when lid 32 is in a closed position. A latch (not shown) is provided for latching and securing lid 32 in a closed position during a sterilization cycle. Cavity 24 defines sterilization chamber 36 when lid 32 is in the closed position.

A fluid circulation system 40 provides for the flow of the liquid sterilant to sterilization chamber 36 and for the circulation of the liquid sterilant in sterilization chamber 36. Fluid circulation system 40 includes a water inlet line 42 that is connected to a source of heated water (not shown). Filter elements 44 and 46 are positioned in water inlet line 42 to filter out large contaminants that may be present in the incoming water. Filters 44 and 46 may comprise size exclusion filter elements used to remove particles exceeding a predetermined size. Filter element 46 may be used to filter out smaller particles than filter element 44. Filter element 44 may be used to filter out particles of about 3 µm (micrometers) or larger, and filter element 46 may be used to filter out particles of about 0.1 µm or larger. Pressure sensors (not shown) may be provided to monitor pressure drops across filter elements 44 and 46. A change in the pressure drop across either filter element may be indicative of clogging, rupturing or the like.

A viral reduction device 52 for inactivating organisms within the water source may be provided in water inlet line 42. Viral reduction device 52 may comprise an ultraviolet (UV) treatment device, for example, a class A device, as specified in NSF/ANSI Standards 55, or an equivalent thereof. An example of such a device would be a UV light system having a minimum dosage of 40,000 µW/cm² which may be available from Wedeco Ideal Horizons of Charlotte, North Carolina. The viral reduction device 52 may be positioned downstream from filter elements 44 and 46, as shown in Fig. 1. Alternatively, the viral reduction device 52 may be positioned in water inlet line 42 upstream of the filter elements 44 and 46.

Water valve 54 may be used to control the flow of water from water inlet line 42 to system feeder line 62. System feeder line 62 includes filtration system 100 to filter out microscopic organisms and particles from the incoming water and thereby provide a sterile water supply to the fluid circulation system 40. System feeder line 62 splits into a first branch feeder line 64 and a second branch feeder line 66. First branch feeder line 64 is connected to container 26 within chamber 36. Second branch feeder line 66 is connected to chamber 36. Secondary branch feeder line 68 splits off of first branch feeder line 64 and is connected to the inlet portion of chemical delivery dispensing container 72. Dispensing container 72 contains components (A) and (B) which, when combined with water, form the liquid sterilant used in the sterilization chamber 36. Valve 74 controls the flow through first branch feeder line 64 and through secondary branch feeder line 68. Chemical dispensing container 72 is positioned within well 76 which is formed within panel 22. Flow restrictors 78 in second branch feeder line 66 and secondary branch feeder line 68 regulate fluid flow through these lines.

Branch return line 82 extends from chemical dispensing container 72 and is connected to system return line 88. Likewise, branch fluid return lines 84 and 86 extend from container 26 and chamber 36, respectively, and are connected to system return line 88. System return line 88 connects back with water inlet line 42 and fluid feeder line 62. Pump 92 is positioned in the system return line 88 and is used to circulate fluid through the fluid circulation system 40. Drain line 94 is connected to system return line 88. Drain valve 96 controls fluid flow to drain line 94.

Referring to Fig. 2, water filtration system 100 is positioned within fluid feeder line 62 and includes filter elements 114 and 134, shown as part of filter assemblies 110 and 130, respectively. First filter assembly 110 includes housing 112 and filter element 114. Second filter assembly 130 includes housing 132 and filter element 134. Filter elements 114 and 134 are positioned in series in fluid feeder line 62. A first section 62a of fluid feeder line 62 connects water inlet line 42 to the inlet side of first filter assembly 110. A second section 62b of fluid feeder line 62 connects the outlet side of first filter assembly 110 to the inlet side of second filter assembly 130. A third section 62c of fluid feeder line 62 connects the outlet side of second filter assembly 130 to heater 102.

Filter elements 114 and 134 may be bacterial retentive size exclusion filters. These may be used to filter out mycobacterium particles having particle sizes that are nominally about 0.12 µm or greater. Filter elements 114 and 134 may include a cylindrical support layer (not shown) made of material such as a polypropylene, surrounded by a filter membrane, such as a hydrophilic polyvinylidene difluoride (PVDF) or a polyethersulfone (PES) filter membrane. The filter membrane may be in the form of a capillary tube or hollow fiber member (or "fiber"), or in the form of a tubular sheath of a film formed either on the inner or outer surface of a tubular macroporous support, or a laminate sheet or film, or a laminate film deposited on the porous support. Suitable filter elements may be obtained from PTI Technologies of Oxnard, California.

Filter element 114 includes an annular outer chamber 116 and inner chamber 118. Outer chamber 116 comprises the upstream, pre-filtration side of filter element 114, and inner chamber 118 represents the downstream, filtered side of filter element 114. First section 62a of fluid feeder line 62 communicates with outer chamber 116, and second section 62b of feeder line 62 communicates with inner chamber 118. A drain line 122 communicates with outer chamber 116. Valve 124 is positioned in drain line 122 to regulate flow from the first filter assembly 110 to a drain.

Filter element 134 includes an annular outer chamber 136 and inner chamber 138. Outer chamber 136 comprises the upstream, pre-filtration side of filter element 134, and the inner chamber 138 represents the downstream, filtered side of filter element 134. Second section 62b of feeder line 62 communicates with outer chamber 136. Third section 62c of feeder line 62 communicates with inner chamber 138. Drain line 142 communicates with outer chamber 136 of second filter assembly 130. Valve 144 is positioned in drain line 142 to regulate flow from second filter assembly 130 to a drain.

The first and second filter assemblies 110 and 130 may be pre-sterilized prior to installation so that the contents of the filter assemblies 110 and 130 may be free of microbial contaminants. The filter assemblies 110 and 130 may be sterilized during each subsequent processing phase.

Valves 152 and 154 are positioned in fluid feeder line 62 to enable isolation of the first filter assembly 110. Valve 152 is positioned within first section 62a of fluid feeder line 62 at the inlet side of first filter assembly 110, and valve 154 is positioned in feeder line section 62b at the outlet side of first filter assembly 110. Similarly, valves 162 and 164 are positioned in fluid feeder line 62 to enable isolation of second filter assembly 130. Valve 162 is positioned in fluid line section 62b at the inlet side of second filter assembly 130, and valve 164 is positioned in fluid feeder line section 62c at the outlet side of second filter assembly 130.

A filter bypass line 172 is connected to fluid feed line 62 on opposite sides of the first and second filter assemblies 110 and 130. One end of bypass line 172 is connected to fluid feed line 62 between pump 92 and the location where the water inlet line 42 connects to fluid feed line 62. A directional check valve 174 is positioned between water inlet line 42 and filter bypass line 172 to prevent incoming water from entering filter bypass line 172. The other end of filter bypass line 172 is connected to feeder line 62 downstream of the filter assemblies 110 and 130, and the heater 102.

Filter purge manifold system 180, which includes air inlet line 182 and vent line 188, may be used to provide clean, filtered, pressurized air to the circulation system 40. Control valve 184 is positioned within air inlet line 182 to regulate the flow of air therethrough. The air in air inlet line 182 may be operated at a predetermined, regulated pressure. Air inlet line 182 may include a pressure regulator (not shown) for maintaining a generally constant, desired air pressure within air inlet line 182. Air inlet line 182 splits into two branch return lines 192 and 194. A vent line 188 with control valve 189 is connected to branch lines 192 and 194. Vent line 188 may be used to allow release of air from the water filtration system 100 during a fill cycle.

First branch line 192 extends through the housing 112 of first filter assembly 110 and communicates with outer chamber 116 of first filter assembly 110. Control valve 196 in first branch line 192 regulates the flow of air therethrough. Second branch line 194 extends through housing 132 of the second filter assembly 130 and communicates with outer chamber 136 of the second filter assembly 130. A control valve 198 is positioned within branch line 194 to regulate flow therethrough.

A first pressure sensor 202 is provided across the first section 62a of system feeder line 62 and branch line 192 to sense pressure on the upstream side of filter element 114.

A second pressure sensor 204 is provided across the second section 62b of system feeder line 62 and branch line 194 to sense pressure on the upstream side of filter element 134.

A first leak orifice line 212 is connected to first section 62a of fluid feed line 62 between the water inlet valve 54 and valve 152 on the upstream side of the first filter assembly 110. A valve 214 within leak orifice line 212 regulates flow therethrough. A flow restrictor 215 is positioned in leak orifice line 212 to regulate flow therethrough.

A second leak orifice line 216 is connected to second section 62b of fluid feed line 62 between valve 154 on the outlet side of first filter assembly 110 and valve 162 on the inlet side of second filter assembly 130. Valve 218 within leak orifice 216 regulates flow therethrough. A flow restrictor 219 is positioned in leak orifice line 216 to regulate flow therethrough.

A drain line 232 is connected to section 62b of system feeder line 62 on the downstream side of filter element 114. A valve 234 regulates flow therethrough. A drain line 236 is connected to section 62c of system feeder line 62 on the downstream side of filter element 134. A valve 238 regulates flow therethrough.

A system microprocessor (not shown) may be used to control the operation of circulation system 40 and the valves therein. The operation of circulation system 40 includes a water fill phase, a chemical generation and sterilization phase, a drain phase, one or more rinse phases, and a filter check phase.

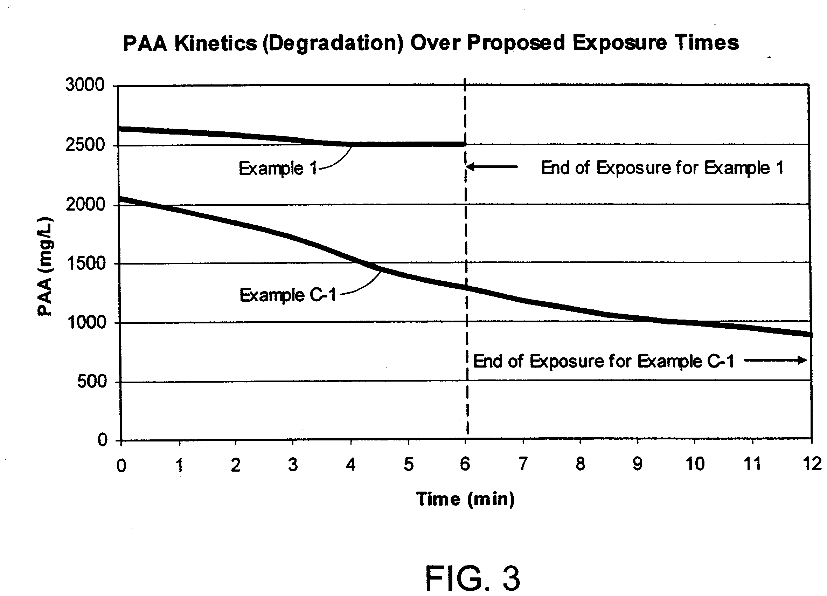

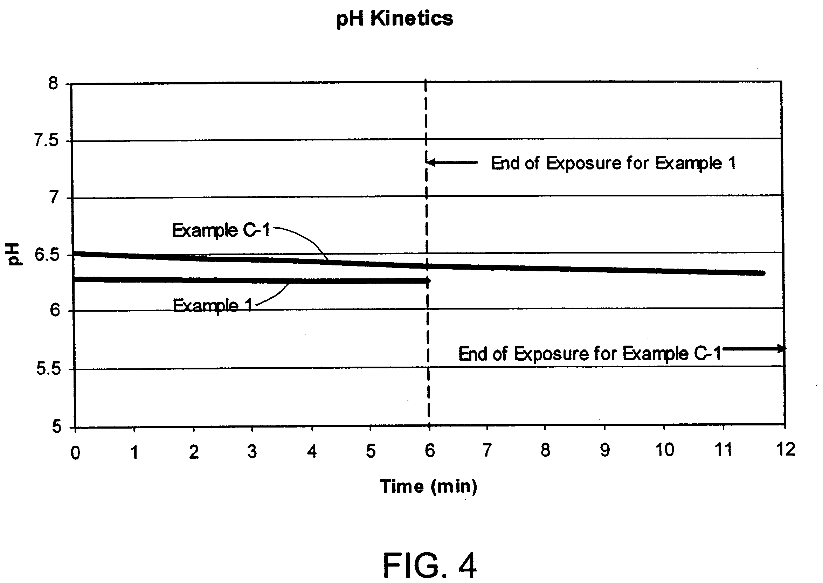

Alternate embodiments of the water filtration system 100 that may be used are disclosed in U.S. Patent 7,569,182 B2, at column 12, line 43 to column 13, line 46, and Figs. 3 and 4.

A sterilization process may be conducted using the apparatus 10 as follows. One or more articles to be sterilized (e.g., medical, dental, pharmaceutical, veterinary or mortuary instruments or devices) are loaded into container 26, which in turn is placed into chamber 36. The articles may be supported on a tray, or in a basket, or a cartridge, or the like (not shown), within the container 26.

The articles may be sterilized using a liquid sterilant formed from water and components (A) and (B). Components (A) and (B) are placed in the chemical dispensing device 72 and contacted with incoming water to form the liquid sterilant. At the beginning of a sterilization process, drain valve 96 in circulation system 40 is closed, and water valve 54 in inlet line 42 is opened to allow heated water to enter circulation system 40. The temperature of the water is in the range from 20 to 80°C, or from 40 to 60°C. The incoming water is filtered using filter elements 44 and 46 in water inlet line 42 to remove particulates greater than a predetermined size. The water may be treated by using a viral reduction device 52 wherein ultraviolet (UV) radiation is applied to the water to inactivate organisms therein. The water passes through valve 54 and enters circulation system 40. The incoming water is filtered using filter assemblies 110 and 130 in feeder line 62 and proceeds to fill the circulation system 40, sterilization chamber 36 and container 26.

Check valve 174 between water inlet valve 54 and filter bypass line 172 causes all of the incoming water to flow through the first and second filter assemblies 110 and 130, thereby insuring filtration of the water flowing into apparatus 10.

The incoming water, which is under pressure from an external source, forces air in the fluid circulation system 40, sterilization chamber 36 and container 26 to an over-flow/air device (not shown) that may be positioned at the highest point of apparatus 10. Air within the system migrates toward the over-flow device.

The presence of the water flowing through the over-flow block is indicative that apparatus 10 is filled with water. The system controller then causes water valve 54 to close, thereby stopping the flow of water into apparatus 10, i.e., into fluid circulation system 40, sterilization chamber 36 and container 26. This completes the water fill phase of the process.

Once the apparatus 10 is filled with water, the system controller initiates the chemical mixing and exposure phase of the process. Pump 92 is energized to circulate water through circulation system 40, sterilization chamber 36 and container 26. Valve 74 is opened to initiate the flow of water through the chemical dispensing container 72. The water and chemical reagents (i.e., components (A) and (B)) positioned in the chemical dispensing container 72, combine to form the liquid sterilant. The liquid sterilant flows into circulation system 40, wherein it is circulated through circulation system 40, sterilization chamber 36 and container 26 by pump 92. A portion of the liquid sterilant flows into sterilization chamber 36 around container 26, and a portion of the liquid sterilant flows into and through container 26 and contacts the articles contained therein.

As indicated by the arrows in Fig. 2, a portion of the circulated liquid sterilant flows through filter bypass line 172 and a portion of the liquid sterilant flows through feed line 62 and the filter assemblies 110 and 130. The amount of fluid flowing through the respective portions of the system may be controlled by regulating valve 222. The portion of the liquid sterilant flowing through filter feed line 62 and through the first and second filter assemblies 110 and 130 should be sufficient to insure sterilization of the filter elements 114 and 134 by exposure to the liquid sterilant. In this respect, the flow of the liquid sterilant through filter assemblies 110 and 130 sterilizes filter elements 114 and 134 and inactivates any microbial contamination that may have entered into filter assemblies 110 and 130 during the water fill phase. During each operation of apparatus 10, filter elements 114 and 134 may be exposed to liquid sterilant and as a result be sterilized by the sterilant. Moreover, the liquid sterilant that flows throughout the closed-loop, fluid circulation system 40 during a sterilization phase, effectively sterilizes the fluid circulation system 40, and the components and fluid conduits forming the same. In other words, fluid circulation system 40 is sterilized during each sterilization cycle.

After a predetermined exposure period, the drain phase may be initiated. The length of the exposure period may range from 0.5 to 240 minutes, or from 2 to 60 minutes. To initiate the drain phase, drain valve 96 is opened and the liquid sterilant is drained from the circulation system 40, sterilization chamber 36 and container 26.

After the liquid sterilant has been drained from the apparatus 10, one or more rinsing phases is performed to rinse any liquid sterilant and any residual matter from the sterilized articles. In this respect, inlet valve 54 is opened to introduce fresh water into apparatus 10, in a manner as heretofore described as the fill phase. All incoming water passes through the water filtration system 100, wherein water entering the circulation system 40 and sterilization chamber 36 is sterile. After each rinse fill, the rinse water is drained from apparatus 10 as heretofore described. Pump 92 may be activated to circulate the rinse water through apparatus 10. During each fill, circulation and drain phase, the fluid over-flow/air make-up assembly operates to prevent microbial contaminants from entering the internal environment within the system. The sterilized article may then be removed from the sterilization chamber.

### Examples

A liquid sterilant is formed by dissolving components (A) and (B) identified in the table below under the heading "Example 1" in processed tap water. The concentration of component (A) is 5.0 grams per liter (g/l), and the concentration of component (B) is 7.7 g/l. This liquid sterilant is representative of the invention.

For purposes of comparison, another liquid sterilant is formed by dissolving components (A) and (B) from the table below under the heading "Example C-1" in tap water. The concentration of component (A) is 5.0 g/l, and the concentration of component (B) is 12.1 g/l. This liquid sterilant is representative of the prior art.

Component (A) is the same for both Examples 1 and C-1. Component (B) for each example is different. Component (B) for Example 1 consists of a relatively simple mixture containing three ingredients, while component (B) for Example C-1 consists of a relatively complex mixture containing twelve ingredients. Also, the weight ratio of component (A) to component (B) is higher for Example 1 than for Example C-1.

| **Ingredients** | **Example 1** | **Example C-1** |
|---|---|---|
| **Component (A):** | | |
| Peracetic Acid 35% | 5.0 g/l | 5.0 g/l |

| **Component (B):** | | |
|---|---|---|
| Three component builder formulation containing disodium phosphate (82.6 wt%), a 40% sodium benzotriazole solution (8.2 wt%) and ethylene diamine tetracetic acid, tetrasodium salt, tetrahydrate (9.2 wt%), characterized by the absence of molybdate | 7.7 g/l | -- |
| Twelve component builder formulation containing two sodium phosphates, benzotriazole, ethylene diamine tetracetic acid, tetrasodiums salt, tetrahydrate, and sodium molybdate (1-10 wt%) | ---- | 12.1 g/l |

| **Weight ratios for Components (A) and (B):** | | |
|---|---|---|
| (A)/(A)+(B) | 0.394 | 0.292 |
| (B)/(A)+(B) | 0.606 | 0.708 |
| (A)/(B) | 0.650 | 0.413 |

There are problems with the prior art, as represented by Example C-1, which are overcome with the inventive composition, as represented by Example 1. These include:
(1) The Example C-1 formulation contains a molybdate. Molybdates are known for protecting white metals from oxidative damage. However, molybdates have been identified as chemical pollutants in many municipal water treatment guidelines, with some municipal governments expressing zero tolerance for their presence in waste streams.
(2) The Example C-1 formulation is complex in that component (B) of the formulation contains twelve ingredients. The use of such a complex formulation results in the requirement for correspondingly complex production and blending methods, and provides for unfavorable interactions between individual ingredients (e.g., caking and concretions). The Example C-1 formulation presents greater difficulties from a quality control perspective than the Example 1 formulation.
(3) The Example C-1 formulation requires greater diligence in tracking the fate and distribution of the various ingredients in the extractables of processed articles or devices and in the subsequent waste water stream.
(4) The Example C-1 formulation is more costly than the Example 1 formulation.
(5) The Example C-1 formulation hardens sooner than the Example 1 formulation under normal storage, transport and use conditions.
(6) The Example C-1 formulation may not be suitable for use in a process employing a 'flow-able' filter wherein sterilization on both sides of the filter is expected to be a necessary prerequisite for clearance of the chemistry and process by the U.S. Food and Drug Administration (FDA). The Example 1 formulation can be used in such a process.

It had been assumed in the prior art that because of the multiple materials and complex designs used in the construction of modern medical, dental, pharmaceutical, veterinary and mortuary instruments, devices, and the like, (e.g., endoscopes), as well as the flux in pH that would be expected under normal use conditions, and the broad range of water hardness that would occur in locations where these sterilization procedures were likely to be performed, that a complex multicomponent liquid sterilant formulation would be required. As such, component (B) for the Example C-1 formulation contains twelve ingredients.

Although there is no doubt about the safety and efficacy of the Example C-1 formulation, it became necessary to modify this formulation in order to provide for its use with flow-able filters in anticipation of new requirements being issued by the FDA. The problem therefore was to provide a replacement formulation in order to comply with the anticipated FDA requirements and at the same time not sacrifice safety or efficacy. This was achieved with the Example 1 formulation. With the Example 1 formulation, it was discovered that a relatively simple formulation could be used that achieves equivalent and sometimes better performance. This was unexpected.

A number of the ingredients in the Example C-1 formulation have potentially toxic effects at certain concentration levels and this had to be accounted for in the design of the replacement formulation. In developing the Example 1 formulation, concentrations relative to acceptable human contact, device tolerance and environmental limitations had to be considered. The fact that these limitations might change over time also had to be taken into account. For example, certain municipalities have recently expressed concern over the environmental impact of molybdenum in waste water. It thus became desirable to remove molybdates from the formulation.

In testing the effects on efficacy as a result of reductions in the amount of molybdenum used in the Example C-1 formulation, it was discovered that other consequences prevailed as well. For instance, the degradation kinetics of peracetic acid in the absence of molybdenum are significantly altered and the resulting pH of the use dilution is also affected. It was discovered that for the Example 1 formulation: (1) the overall flux in pH over time (kinetics) in the presence of the typical amounts of buffer that would be used would differ significantly from that in the Example C-1 formulation; (2) the degradation of peracetic acid would be effectively eliminated to a degree beyond what might be normally expected; and (3) the net corrosivity would not be unfavorably altered.

It was unexpectedly discovered that a substantial number of the ingredients in component (B) of the Example C-1 formulation could be removed in providing the Example 1 formulation with no apparent unfavorable consequences with respect to compatibility or potency. With the Example 1 formulation, it was initially assumed that in order to continue the use of the 12 minute exposure for sterilization runs, which had been successfully validated for the Example C-1 formulation, it would be necessary to expose the article being sterilized to a higher peracetic acid (PAA) concentration over time. It was thought that this might result in damage to the article being sterilized as a consequence of excessive exposure to harsh sterilizing conditions. However, it was discovered that with the Example 1 formulation that it was possible to achieve potency results equivalent to those achieved with the Example C-1 formulation with a much shorter exposure (i.e., about 6 minutes) and without the expected increase in damage with longer exposure time.

The Example 1 formulation may be regarded as a simplified, single-use oxidative chemistry formulation comprising an active component, i.e., component (A), and a builders component, i.e., component (B). The Example 1 formulation is at least as safe and effective as a germicide as the Example C-1 formulation, and it may be used to sterilize both sides of a flow-able filter as required for submission to the FDA.

The assumption in the prior art had been that a complex formulation such as that provided by Example C-1 is needed in order to balance germicidal efficacy with potential damage to the articles being sterilized. Thus, for example, it had been presumed that a molybdate is needed to protect certain metal components from corrosion caused by the peracetic acid. However, the Example 1 formulation is characterized by the absence of a molybdate and despite this absence, corrosion that was anticipated without the molybdate is not observed. This was unexpected.

An increase in the concentration of active peracetic acid (PAA) in use dilution is observed when the Example 1 formulation is used. This is believed to be attributable to the removal of molybdate from the formulation. The extent of the increase in peracetic acid concentration for the Example 1 formulation as compared to the Example C-1 formulation was unexpected. With the Example C-1 formulation, the initial concentration of peracetic acid diminishes rapidly with time. On the other hand, with the Example 1 formulation the initial concentration of peracetic acid diminishes far less and achieves a nearly constant value over extended periods. This is shown in Fig. 3. While this increase in the active peracetic acid concentration may be advantageous for purposes of bactericidal efficacy, it raises the possibility that too much peracetic acid may cause damage to the articles being sterilized.

The increase in peracetic acid concentration that occurs with the Example 1 formulation was so significant that it was believed to be necessary to offset the resulting imbalance between efficacy and safety that the removal of the molybdate appeared to create. However, rather than reintroducing a molybdate, or another modulating ingredient, the relative proportions of the remaining ingredients were changed to provide the Example 1 formulation. Also, when using the Example 1 formulation to form a liquid sterilant the relative amount of peracetic acid used can be correspondingly decreased and/or the exposure time the article being sterilized is in contact with the sterilant can be correspondingly decreased. By removing the molybdenum, the resulting pH is only marginally changed and is effectively equivalent to that of the Example C-1 formulation without significantly changing the kinetics relating to the improvement in peracetic acid concentration. This is shown in Fig. 4. This indicates that the required balance between the optimal germicidal reactivity (pH 5-7) and optimal device safety (pH 6-8) may be maintained.

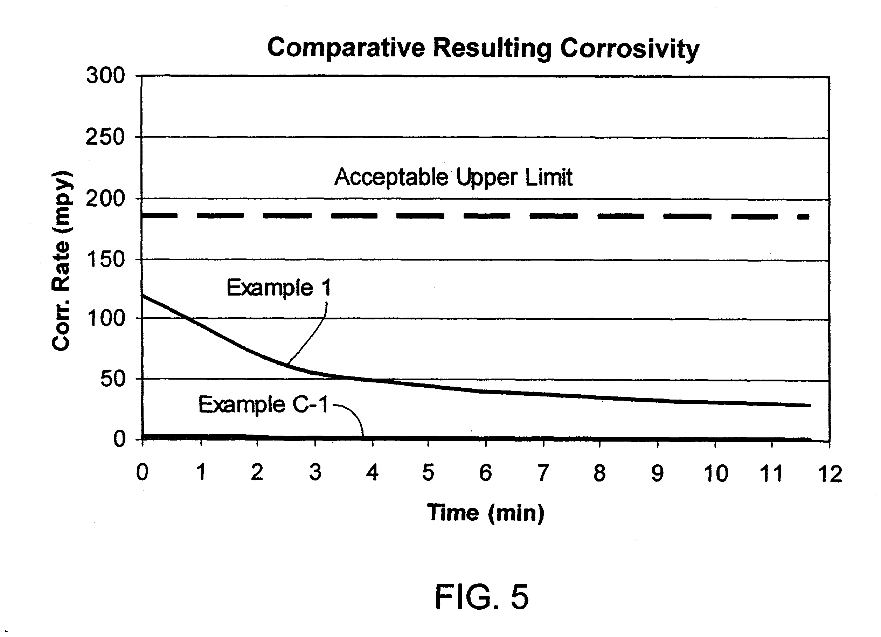

Corrosion testing indicates that the relative resulting corrosivity of the Example 1 formulation, while somewhat higher than that of the Example C-1 formulation, is still at an acceptable level. This is shown in Fig. 5. Thus, with the Example 1 formulation it is possible to attain a significant increase in peracetic acid concentration while maintaining favorable pH and acceptable corrosivity levels.

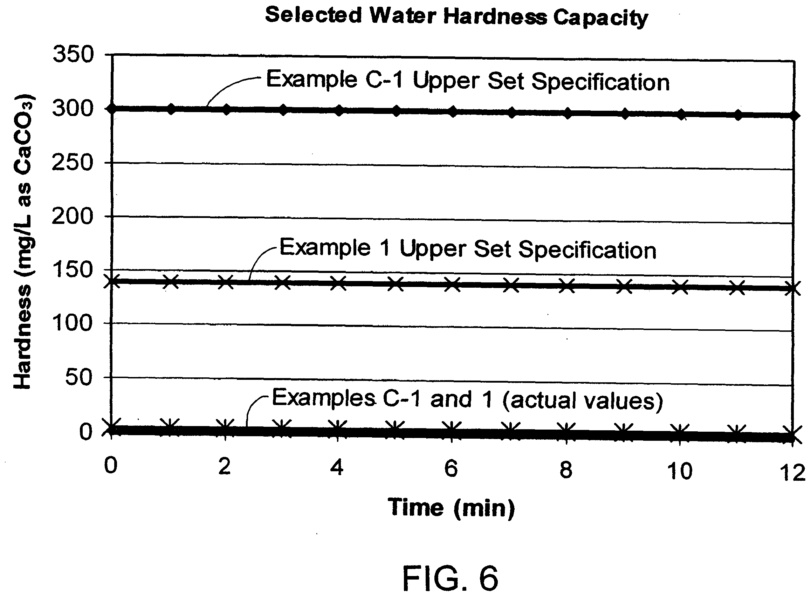

Even though the requirements for the levels of chelation capacity needed for the Example 1 formulation are changed from that required by the original Example C-1 formulation (140 ppm and 300 ppm, respectively), the Example 1 formulation has been adjusted to attain the end point water hardness desired for this new application. This is shown in Fig. 6.

Because of the simplicity of the Example 1 formulation, the dissolution of dry ingredients proceeds faster than with Example C-1 formulation. Thus, for example, in a sterilization using the Example C-1 formulation, a warm/mix phase of 8 minutes may be required while with the Example 1 formulation only 1-3 minutes may be required. Also due to its simplicity, the Example 1 formulation gives rise to a use dilution that is easier to rinse away at the conclusion of the processing cycle as compared to the Example C-1 formulation. The Example C-1 formulation may require 4 rinse cycles to reduce the amount of extractable residues to safe levels while the Example 1 formulation may achieve similar levels after just 2 or fewer rinse cycles. Taken cumulatively, these reductions in time may result in an overall sterilization cycle for the Example 1 formulation that is less than half the length of the cycle needed for the Example C-1 formulation. Thus, with the Example 1 formulation it may be possible to achieve a significant time saving benefit, along with the additional benefit of retaining the balance of safety and efficacy when compared to using the Example C-1 formulation.

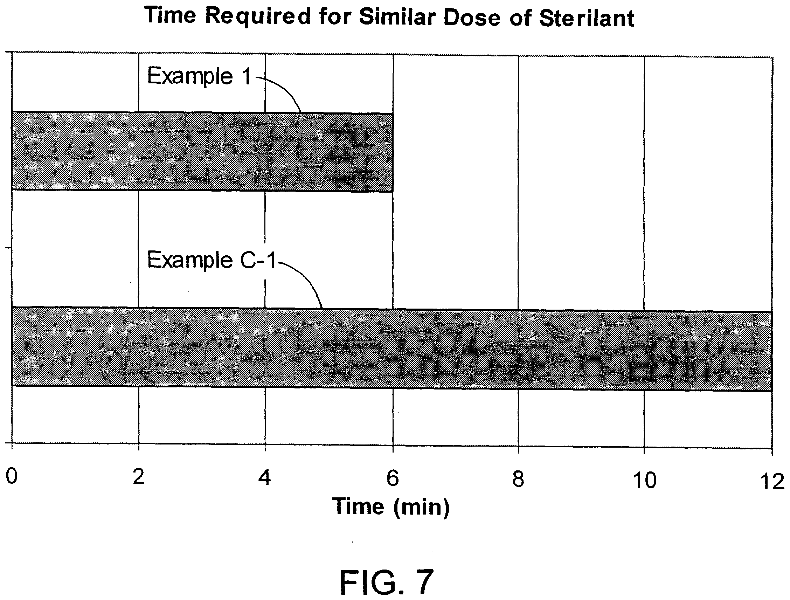

Consequently, and unexpectedly, it may be possible to achieve the same exposure dose (mg/L peracetic acid min⁻¹) of the active ingredient (i.e., peracetic acid) for the Example 1 formulation in less than one-half the time required for the Example C-1 formulation. This relationship is shown in Fig. 7.

The advantages of using the Example 1 formulation as compared to the Example C-1 formulation include:
(1) The Example 1 formulation provides a higher total concentration (mg/mL) of peracetic acid throughout the cycle which enables a shorter overall cycle time while maintaining the equivalent dose. See, Figs. 3 and 7.
(2) The Example 1 formulation is characterized by the absence of a molybdate, which is advantageous from an environmental perspective. In fact the Example 1 formulation contains no material that is currently (at its proposed concentration) non-compliant with any environmental watch list.
(3) The Example 1 formulation is simple. It contains only those ingredients found to be necessary to achieve the desired functions. This allows for a much simpler production and blending program with easier quality control measures, and a simpler analysis for all ingredients.
(4) The Example 1 formulation enables the reduction of required rinse cycles needed from four to two or fewer thus saving time in the cycle and utility costs for the customer. At over 11 million cycles per year (which is the anticipated market use for the Example 1 formulation), this translates to a savings of approximately 60 million gallons of municipally treated water used per year.
(5) There is no evidence that any of the ingredients in the Example 1 formulation interact with each other in any way other than to support safety and efficacy.
(6) The Example 1 formulation features a far less complex formulation than the Example C-1 formulation and thus far less diligence is required in tracking the fate and distribution of its ingredients in the extractables of sterilized articles or in the subsequent waste stream.
(7) The Example 1 formulation is comprised of fewer ingredients that are easier to source, that are made by multiple vendors, and are easier to control with respect to their more common specifications.
(8) The Example 1 formulation employs the use of less expensive ingredients and with fewer total ingredients which reduces the overall material costs.
(9) The Example 1 formulation provides for better overall shelf life and stability with a reduced tendency to cake or harden as often happens with the Example C-1 formulation.
(10) The Example 1 formulation provides for more rapid and effective dissolution in water to form a liquid sterilant.
(11) The Example 1 formulation provides for a substantially shorter exposure time (less than or equal to 6 minutes for the Example 1 formulation vs. 12 minutes for the Example C-1 formulation).

## Claims

1. Method for sterilizing a medical, dental, pharmaceutical, veterinary or mortuary instrument at a temperature of 20°C to 8o°C using a liquid sterilant, comprising supplying components (A) and (B) separately and dispersing the supplied components (A) and (B) in water at the time the sterilizing is conducted, wherein:
component (A) consists essentially of: 15 to 45% by weight peracetic acid; 34 to 62% by weight acetic acid; 6.5 to 32% by weight hydrogen peroxide; and 0.5 to 2% by weight sulfuric acid; and
component (B) consists essentially of: 35 to 98% by weight of a buffer, wherein the buffer is selected from the group consisting of an alkali metal phosphate, an alkali metal carbonate, or a mixture thereof; 0.5 to 35% by weight of an anticorrosive agent; and 0.1 to 60% by weight of a chelator; and
wherein the weight ratio of component (A) to component (B) is from 0.1 to 1.3; and wherein the component (B) comprises molybdate, nonylphenol ethoxylate, and an antifoaming agent, respectively in an amount of 0.01% by weight or less.

2. Method according to claim 1, wherein the concentration of component (A) in the aqueous liquid sterilant is in the range from 0.5 to 10 g/l, and the concentration of component (B) in the aqueous liquid sterilant is in the range from 3.6 to 18 g/l, and the liquid sterilant has a pH in the range from 2 to 11, or from 5.5 to 7.

3. Method according to claim 1 or 2 further comprising contacting the medical, dental, pharmaceutical, veterinary or mortuary instrument with the liquid sterilant.

4. Method according to any of the preceding claims, wherein the exposure time of the medical, dental, pharmaceutical, veterinary or mortuary instrument is in the range from 0.5 to 240 min, or from 2 to 60 min.

## Patentansprüche

1. Verfahren zum Sterilisieren eines medizinischen, zahnmedizinischen, pharmazeutischen, tiermedizinischen oder leichenmedizinischen Instruments bei einer Temperatur von 20°C bis 80°C unter Verwendung eines flüssigen Sterilisationsmittels, umfassend ein separates Liefern von Komponenten (A) und (B) und Dispergieren der gelieferten Komponenten (A) und (B) in Wasser zu dem Zeitpunkt, wo das Sterilisieren durchgeführt wird, wobei:
Komponente (A) im Wesentlichen besteht aus: 15 bis 45 Gew.-% Peressigsäure; 34 bis 62 Gew.-% Essigsäure; 6,5 bis 32 Gew.-% Wasserstoffperoxid; und 0,5 bis 2 Gew.-% Schwefelsäure; und
Komponente (B) im Wesentlichen besteht aus: 35 bis 98 Gew.-% eines Puffers, wobei der Puffer ausgewählt wird aus der Gruppe bestehend aus einem Alkalimetallphosphat, einem Alkalimetallcarbonat oder einer Mischung derselben; 0,5 bis 35 Gew.-% eines antikorrosiven Mittels; und 0,1 bis 60 Gew.-% eines Chelators; und
wobei das Gewichtsverhältnis von Komponente (A) zu Komponente (B) von 0,1 bis 1,3 ist; und wobei die Komponente (B) Molybdat, Nonylphenolethoxylat bzw. ein Antischäumungsmittel in einer Menge von 0,01 Gew.-% oder weniger umfasst.

2. Verfahren nach Anspruch 1, wobei die Konzentration der Komponente (A) in dem wässrigen flüssigen Sterilisationsmittel im Bereich von 0,5 bis 10 g/l ist, und die Konzentration der Komponente (B) in dem wässrigen flüssigen Sterilisationsmittel im Bereich von 3,6 bis 18 g/l ist, und wobei das flüssige Sterilisationsmittel einen pH-Wert im Bereich von 2 bis 11 oder von 5,5 bis 7 aufweist.

3. Verfahren nach Anspruch 1 oder 2, weiter umfassend ein Kontaktieren des medizinischen, zahnmedizinischen, pharmazeutischen, tiermedizinischen oder leichenmedizinischen Instruments mit dem flüssigen Sterilisationsmittel.

4. Verfahren nach einem der vorangehenden Ansprüche, wobei die Expositionszeit des medizinischen, zahnmedizinischen, pharmazeutischen, tiermedizinischen oder leichenmedizinischen Instruments im Bereich von 0,5 bis 240 min oder von 2 bis 60 min ist.

## Revendications

1. Procédé de stérilisation d'un instrument médical, dentaire, pharmaceutique, vétérinaire ou mortuaire à une température de 20 °C à 80 °C en utilisant un agent stérilisant liquide, comprenant la fourniture des composants (A) et (B) séparément et la dispersion des composants (A) et (B) fournis dans de l'eau au moment où la stérilisation est effectuée, dans lequel :
le composant (A) consiste essentiellement de 15 à 45 % en poids d'acide paracétique ; de 34 à 62 % en poids d'acide acétique ; de 6,5 à 32 % en poids de peroxyde d'hydrogène et de 0,5 à 2 % en poids d'acide sulfurique ; et
le composant (B) consiste essentiellement de : 35 à 98 % d'un tampon, dans lequel le tampon est sélectionné dans le groupe constitué d'un phosphate de métal alcalin, d'un carbonate de métal alcalin, ou d'un mélange de ceux-ci ; de 0,5 à 35 % en poids d'un produit anticorrosion et de 0,1 à 60 % en poids d'un chélateur ; et
dans lequel le rapport pondéral du composant (A) au composant (B) s'étend de 0,1 à 1,3 ; et dans lequel le composant (B) comprend respectivement du molybdate, de l'éthoxylate de nonylphénol et un agent antimoussant, en une quantité de 0,01 % en poids ou inférieure.

2. Procédé selon la revendication 1, dans lequel la concentration du composant (A) dans l'agent stérilisant liquide aqueux se situe dans la plage de 0,5 à 10 g/l, et la concentration du composant (B) dans l'agent stérilisant liquide aqueux se situe dans la plage de 3,6 à 18 g(l, et l'agent stérilisant liquide a un pH se situant dans la plage de 2 à 11 ou de 5,5 à 7.

3. Procédé selon la revendication 1 ou 2 comprenant en outre la mise en contact de l'instrument médical, dentaire, pharmaceutique, vétérinaire ou mortuaire avec l'agent stérilisant liquide.

4. Procédé selon l'une quelconque des revendications précédentes, dans lequel le temps d'exposition de l'instrument médical, dentaire, pharmaceutique, vétérinaire ou mortuaire se situe dans la plage de 0,5 à 240 minutes ou de 2 à 60 minutes.
